# EUROPÄISCHE PATENTSCHRIFT

(11) **EP 3 792 337 B1**
(45) Veröffentlichungstag und Bekanntmachung des Hinweises auf die Patenterteilung: **07.02.2024**
(21) Anmeldenummer: 20174367.1
(22) Anmeldetag: 13.05.2020
(51) Int. Cl.: C10G 25/00, C07C 7/12, C10G 25/05, C10G 25/12

(54) **VERFAHREN ZUR REINIGUNG EINES KOHLENWASSERSTOFFSTROMS MIT RESTBUTEN**
METHOD FOR PURIFYING A HYDROCARBON FLOW WITH RESIDUAL BUTENE
PROCÉDÉ DE NETTOYAGE D'UN FLUX D'HYDROCARBURES CONTENANT DU BUTÈNE RÉSIDUEL

(30) Priorität: 14.09.2019 EP 19174289
(43) Veröffentlichungstag der Anmeldung: 17.03.2021
(73) Patentinhaber: Evonik Oxeno GmbH & Co. KG, 45772 Marl (DE)
(72) Erfinder: Peitz, Stephan, 45739 Oer-Erkenschwick (DE); Stochniol, Guido, 45721 Haltern am See (DE); Müller, Anja, 70563 Stuttgart (DE); Moeller, Oliver, 45739 Oer-Erkenschwick (DE)
(74) Vertreter: Evonik Patent Association

(56) Entgegenhaltungen:
- WO-A2-2013/013885
- US-A1- 2015 025 284
- US-A1- 2017 247 298

## Beschreibung

Die vorliegende Erfindung betrifft ein Verfahren zur Reinigung von Kohlenwasserstoffströmen von Stickstoff-, Sauerstoff- und/oder Schwefel-haltige Verunreinigungen vor dem Einsatz in einem Olefinumsetzungsverfahren und die Verwendung der gereinigten Kohlenwasserstoffströme.

In der petrochemischen Industrie werden häufig Kohlenwasserstoffströme, die leichtere Olefine mit bis zu 8 Kohlenstoffatomen enthalten, als Einsatzströme für chemische Prozesse, wie die Veretherung, die Hydroformylierung, die Isomerisierung, die Oligomerisierung, die Alkylierung, die Metathese, die Epoxidation oder die (Methoxy)Carbonylierung eingesetzt. Die eingesetzten Kohlenwasserstoffströme sind üblicherweise technische Kohlenwasserstoffgemische, die beispielsweise aus Steamcrackern oder katalytischen Crackern stammen. Diese Kohlenwasserstoffgemische enthalten üblicherweise Stickstoff-, Sauerstoff und/oder Schwefel-haltige Verunreinigungen im ppm-Bereich, die als Katalysatorgifte für die bei den vorgenannten chemischen Prozessen eingesetzten Katalysatoren wirken können.

Die Gegenwart der genannten Verunreinigungen kann aufgrund deren katalysatorschädigender Wirkung, beispielsweise Desaktivierung durch Adsorption der Verunreinigungen auf dem Katalysator und der daraus resultierenden Blockierung der aktiven Zentren und Poren des Katalysators, einen erheblichen negativen Einfluss auf die Lebensdauer des jeweiligen Katalysators und damit auch auf die Durchführung und die Wirtschaftlichkeit des entsprechenden chemischen Prozesses haben. Um diese Probleme zu vermeiden, sollten die Stickstoff-, Sauerstoff- und/oder Schwefel-haltige Verunreinigungen vor dem Durchlaufen der genannten chemischen Prozesse entfernt werden.

Die Entfernung von Stickstoff-, Sauerstoff- und/oder Schwefel-haltige Verunreinigungen aus Kohlenwasserstoffströmen ist an sich bekannt. Durch die Verwendung spezieller Adsorber sollen Verunreinigungen so ausreichend aus den Kohlenwasserstoffströmen entfernt werden, dass eine Vergiftung des Katalysators für den nachgeschalteten chemischen Prozess verzögert oder vollständig verhindert wird.

Die für die Entfernung der Verunreinigungen eingesetzten Adsorber haben nur eine begrenzte Kapazität für die Aufnahme der Verunreinigungen. Anstatt die gebrauchten Adsorber gegen neue Adsorber auszutauschen, ist es gängige Praxis die Adsorber zu regenerieren, beispielsweise mittels eines Inertgasstroms, der über den gebrauchten Adsorber geleitet wird.

Ein weiteres Verfahren zur Regeneration eines solchen gebrauchten Adsorbers, der einer Oligomerisierung vorgeschaltet ist, ist aus der WO 2013/013885 A2 bekannt. Dabei wird der Produktstrom einer Oligomerisierung über den gebrauchten und zu regenerierenden Adsorber geleitet, um diesen von adsorbierten Verunreinigungen zu befreien. Nach erfolgter Regeneration kann der regenerierte Adsorber erneut eingesetzt werden, um Verunreinigungen aus dem Einsatzstrom zu entfernen.

Das Problem bei den bekannten Verfahren ist, dass Inertgas vergleichsweise teuer ist und nicht an jedem Produktionsstandort ohne größeren Aufwand in den benötigen Mengen bereitgestellt werden kann. Die Verwendung von Produktströmen aus nachgeschalteten chemischen Prozessen, wie der Oligomerisierung, zur Reinigung des Kohlenwasserstoffstroms hat den Nachteil, dass der Produktstrom durch die Desorption der Verunreinigungen kontaminiert würde, wodurch die Nutzung und/oder der Verkauf der Produkte erschwert wird.

Die Aufgabe der vorliegenden Erfindung war deshalb die Bereitstellung eines einem chemischen Prozess vorgelagerten Verfahrens, mit dem ein gebrauchter Adsorber regeneriert werden kann, ohne dass die vorgenannten Nachteile auftreten.

Die zugrundeliegende technische Aufgabe konnte durch das in Anspruch 1 beschriebene Verfahren gelöst werden. Bevorzugte Ausführungsformen sind in den Unteransprüchen angegeben.

Der Gegenstand der vorliegenden Erfindung ist mithin ein Verfahren zur Reinigung von Kohlenwasserstoffströmen, umfassend Olefine mit 2 bis 8 Kohlenstoffatomen, wobei der gereinigte Kohlenwasserstoffstrom mindestens einem Verfahrensschritt, welcher eine Olefinumsetzung, welche aus der Gruppe, bestehend aus Veretherung, Hydroformylierung, Oligomerisierung, (Methoxy)Carbonylierung, Alkylierung, Metathese und Epoxidation, ausgewählt wird, und eine Produktabtrennung umfasst, unterworfen wird,
wobei in dem Verfahren mindestens zwei Adsorber verwendet werden, von denen sich ein Adsorber im Adsorptionsmodus und ein Adsorber im Desorptionsmodus befindet,
wobei im Adsorptionsmodus der zu reinigende Kohlenwasserstoffstrom über den Adsorber geleitet wird, um Stickstoff-, Sauerstoff- und/oder Schwefel-haltige Verunreinigungen aus dem zu reinigenden Kohlenwasserstoffstrom zu entfernen,
wobei im Desorptionsmodus ein Desorptionsstrom bei einer Temperatur von 280 bis 350 °C über den Adsorber geleitet wird, um die adsorbierten Verunreinigungen vom Adsorber zu desorbieren, und
dadurch gekennzeichnet, dass ein nach dem mindestens einen Verfahrensschritt entnommener und an den eingesetzten Olefinen und den bei der Olefinumsetzung gebildeten Produkten abgereicherter Kohlenwasserstoffstrom, der nicht hydriert wird und der einen Gehalt an den eingesetzten und nicht umgesetzten Olefinen von 2 bis 30 Gew.-% aufweist, als Desorptionsstrom verwendet wird.

Das erfindungsgemäße Verfahren zur Reinigung betrifft Kohlenwasserstoffströme, die Olefine mit 2 bis 8 Kohlenstoffatomen, vorzugsweise 3 bis 6 Kohlenstoffatomen, besonders bevorzugt 4 Kohlenstoffatomen umfassen. Geeignete Olefine sind unter anderem α-Olefine, n-Olefine und Cycloalkene, vorzugsweise n-Olefine. In einer bevorzugten Ausführungsform handelt es sich bei dem Olefin um n-Buten.

Die Olefine werden üblicherweise nicht in reiner Form als Edukte eingesetzt, sondern in technisch verfügbaren Mischungen. Der in dieser Erfindung zusätzlich verwendete Begriff Kohlenwasserstoffstrom ist deshalb so zu verstehend, dass er jegliche Art von Gemischen betrifft, die die entsprechenden Olefine in einer Menge enthalten, die es ermöglicht, die möglichen der Reinigung nachgeschalteten Verfahrensschritte wirtschaftlich durchzuführen. Der eingesetzte Kohlenwasserstoffstrom weist daher vorzugsweise einen Gehalt an Olefinen von 35 bis 95 Gew.-%, vorzugsweise von 70 bis 95 Gew.-% auf.

Der nach dem mindestens einen Verfahrensschritt entnommene und an den eingesetzten Olefinen und den bei der Olefinumsetzung gebildeten Produkten abgereicherte Kohlenwasserstoffstrom weist einen Gehalt an den eingesetzten und nicht umgesetzten Olefinen von 2 bis 30 Gew.-% auf, da ein signifikanter Teil der eingesetzten Olefine in dem Umsetzungsverfahren des mindestens einen Verfahrensschritt zu den jeweiligen Produkten reagiert worden ist. Aufgrund der sich anschließenden Produktabtrennung sind vorzugsweise nur geringe Mengen an Produkten im resultierenden Kohlenwasserstoffstrom vorhanden. In einer bevorzugten Ausführungsform weist der nach dem mindestens einen Verfahrensschritt entnommene und an den eingesetzten Olefinen und den bei der Olefinumsetzung gebildeten Produkten abgereicherte Kohlenwasserstoffstrom einen Gehalt an Produkten von maximal 500 Gewichts-ppm, weiterhin bevorzugt maximal 300 Gewichts-ppm, besonders bevorzugt maximal 170 Gewichts-ppm auf.

Propylen als Olefin mit 3 Kohlenstoffatomen wird großtechnisch durch Spaltung von Naphtha hergestellt und ist eine Grundchemikalie, die leicht verfügbar ist. Olefine mit 5 Kohlenstoffatomen sind in Leichtbenzinfraktionen aus Raffinerien oder Crackern enthalten. Technische Gemische, die lineare Olefine mit 4 Kohlenstoffatomen enthalten, sind Leichtbenzinfraktionen aus Raffinerien, C4-Fraktionen aus FC- oder Steamcrackern, Gemische aus Fischer-Tropsch-Synthesen, Gemische aus der Dehydrierung von Butanen oder durch Metathese oder aus anderen technischen Prozessen entstandene Gemische. Beispielsweise können für das erfindungsgemäße Verfahren geeignete Gemische linearer Butene aus der C4-Fraktion eines Steamcrackers gewonnen werden. Dabei wird im ersten Schritt Butadien entfernt. Dies geschieht entweder durch Extraktion(sdestillation) des Butadiens oder dessen Selektivhydrierung. In beiden Fällen wird ein praktisch butadienfreier C4-Schnitt erhalten, das sogenannte Raffinat I. Im zweiten Schritt wird Isobuten aus dem C4-Strom entfernt, z. B. durch Herstellung von MTBE durch Umsetzung mit Methanol. Der jetzt isobutenfreie und butadienfreie C4-Schnitt, das sogenannte Raffinat II, enthält die linearen Butene und gegebenenfalls Butane. Wird daraus auch noch zumindest ein Teil des enthaltenen 1-Butens abgetrennt, erhält man das sogenannte Raffinat III.

In einer bevorzugten Ausführungsform werden im erfindungsgemäßen Verfahren C4-olefinhaltige Stoffströme als Einsatzgemisch zugeführt. Geeignete Olefingemische sind insbesondere das Raffinat I und das Raffinat II und das Raffinat III.

Die vorgenannten, einsetzbaren Kohlenwasserstoffströme enthalten üblicherweise herstellungsbedingt Verunreinigungen, also Kleinstmengen an anderen Substanzen. Dazu zählen insbesondere Stickstoff-, Sauerstoff- und/oder Schwefel-haltige Verunreinigungen, insbesondere die Stickstoff-haltigen Verunreinigungen Acetonitril und Propionitril, die Sauerstoff-haltigen Verunreinigungen Aceton und Methanol und/oder die Schwefel-haltigen Verunreinigungen Methanthiol, Ethanthiol, Dimethylsulfid und Dimethyldisulfid.

Zur Entfernung der vorgenannten Verunreinigungen, die als Katalysatorgifte wirken können, wird der eingesetzte und zu reinigende Kohlenwasserstoffstrom erfindungsgemäß über einen Adsorber geleitet, auf dem die Verunreinigungen adsorbiert werden. Als Adsorber können Materialien mit ausreichender Adsorptionsfähigkeit und -stabilität verwendet werden, insbesondere Silicate, Aluminiumoxide und zeolithische Materialien wie Zeolith A (z.B. 3A, 4A, 5A), Faujasit (z.B. NaX, 13X, NaY), MFI (z.B. Silicalit-1, ZSM-5), Mordenit, Chabazite (z.B. SSZ-13), Ferrierit, sowie mit adsorptionsaktiven Metallen/Metalloxiden (z.B. Cu, Ni) belegte Varianten der vorgenannten Materialien eingesetzt werden. Es können auch Mischungen der genannten Materialien eingesetzt werden. Die Adsorber können in einem druck- und temperaturbeständigen Behälter, insbesondere einem Reaktor angeordnet sein, durch den der zu reinigende Kohlenwasserstoffstrom geleitet wird.

In dem erfindungsgemäßen Verfahren werden mindestens zwei Adsorber verwendet, die sich jeweils immer in einem unterschiedlichen Modus, entweder dem Adsorptionsmodus oder dem Desorptionsmodus, befinden. In der Ausführungsform mit zwei Adsorbern befinden sich ein Adsorber im Adsorptionsmodus und ein Adsorber im Desorptionsmodus. Die beiden Adsorber sind jeweils ausgewählt aus den vorgenannten Materialien und können gleich oder verschieden voneinander sein.

Der Adsorptionsmodus zeichnet sich dadurch aus, dass der zu reinigende Kohlenwasserstoffstrom über den Adsorber geleitet wird, um Stickstoff-, Sauerstoff- und/oder Schwefel-haltige Verunreinigungen aus dem zu reinigenden Kohlenwasserstoffstrom zu entfernen. In einer bevorzugten Ausführungsform wird der zu reinigende Kohlenwasserstoffstrom in flüssiger Phase über den mindestens einen Adsorber im Adsorptionsmodus geleitet. So lässt sich eine besonders gute Reinigung des Kohlenwasserstoffstroms erreichen. Die Temperatur, bei der der zu reinigende Kohlenwasserstoffstrom über den mindestens einen Adsorber im Adsorptionsmodus geleitet wird, liegt vorzugsweise im Bereich von 0 bis 60 °C, vorzugsweise 10 bis 50 °C, besonders bevorzugt 20 bis 40 °C. Der Druck sollte bei den genannten Temperaturen in dem Fachmann bekannter Weise so eingestellt werden, dass der zu reinigende Kohlenwasserstoffstrom in flüssiger Phase vorliegt.

Im Desorptionsmodus wird erfindungsgemäß ein Desorptionsstrom über den Adsorber geleitet, um die adsorbierten Verunreinigungen vom Adsorber zu desorbieren. In einer bevorzugten Ausführungsform strömt der Desorptionsstrom in entgegengesetzter Richtung zur Strömungsrichtung des zu reinigen Kohlenwasserstoffstroms im Adsorptionsmodus über den Adsorber. Der Adsorber wird dadurch regeneriert und kann nach Entfernung der Verunreinigungen wieder als Adsorber im Adsorptionsmodus insofern eingesetzt werden, als dass der Adsorber nun wieder Verunreinigungen aus dem zu reinigenden Kohlenwasserstoffstrom in ausreichendem Maße aufnehmen kann.

Der Desorptionsstrom ist erfindungsgemäß ein Kohlenwasserstoffstrom, der mindestens einem nachgeschalteten Verfahrensschritt, die jeweils eine Olefinumsetzung, welche aus der Gruppe, bestehend aus Veretherung, Hydroformylierung, Oligomerisierung, (Methoxy)Carbonylierung, Alkylierung, Metathese und Epoxidation, ausgewählt wird, und eine Produktabtrennung umfassen, an den eingesetzten Olefinen abgereichert entnommen worden ist. Der entsprechende Kohlenwasserstoffstrom wird demzufolge nicht hydriert, sodass der Strom weiterhin signifikante Mengen an eingesetzten und nicht umgesetzten Olefinen enthalten kann. In einer weiteren Ausführungsform der vorliegenden Erfindung können auch mehr als ein, also mindestens zwei Verfahrensschritte, die auf die Reinigung durch Adsorption folgen, vorhanden sein. Der Desorptionsstrom ist dann ein Kohlenwasserstoffstrom, der einem oder mehreren der Reinigung nachgeschalteten Verfahrensschritten, die jeweils eine Olefinumsetzung, welches ausgewählt wird aus der Gruppe, bestehend aus Veretherung, Hydroformylierung, Oligomerisierung, (Methoxy)Carbonylierung, Alkylierung, Metathese und Epoxidation, und eine Produktabtrennung umfassen, an den eingesetzten Olefinen abgereichert entnommen worden ist.

Sind mindestens zwei Verfahrensschritte vorhanden, kann der an den eingesetzten Olefinen abgereicherte Kohlenwasserstoffstrom dabei nach einem einzigen Verfahrensschritt (im Falle von zwei Verfahrensschritten beispielsweise dem ersten Verfahrensschritt), nach zwei Verfahrensschritten oder, sofern mehr als zwei Verfahrensschritte vorliegen, nach mehr als zwei Verfahrensschritten entnommen werden. Wird der Kohlenwasserstoffstrom zwei oder mehr Verfahrensschritten entnommen, handelt es sich demnach um eine Mischung der jeweiligen an den eingesetzten Olefinen abgereicherten Ströme. Dadurch wird es ermöglicht, die Ströme variabel einzusetzen.

Der Begriff Verfahrensschritt umfasst dabei im Sinne der vorliegenden Erfindung sowohl die Umsetzung der eingesetzten Olefine als auch eine anschließende Abtrennung der gebildeten Produkte (Produktabtrennung), beispielsweise eine Destillation, eine Membrantrennung, eine Extraktion oder eine Kombination daraus, und optional eine vor der Olefinumsetzung angeordnete Selektivhydrierung von mehrfach ungesättigten Olefinen oder eine vor der Olefinumsetzung angeordnete, vorzugsweise destillative, 1-Olefin-Abtrennung oder Olefin-Paraffin-Trennung. Die Verfahren und deren typische Verfahrensbedingungen sind dem Fachmann geläufig. Der Desorptionsstrom ist also ein Ausgangsstrom aus dem mindestens einem Verfahrensschritt, umfassend eine Olefinumsetzung und eine auf die Olefinumsetzung nachfolgende Produktabtrennung, bei dem die gebildeten Produkte vom restlichen Kohlenwasserstoffstrom, der nach der Produktabtrennung zumindest einen Teil der eingesetzten und nicht umgesetzten Olefine und Alkane umfasst, abgetrennt werden. Bei der Hydroformylierung oder der Oligomerisierung werden beispielsweise nachfolgend die gebildeten Aldehyde bzw. Oligomere von den nicht umgesetzten Olefinen und inerten Alkanen abgetrennt.

In einer weiterhin bevorzugten Ausführungsform wird nach dem mindestens einen Verfahrensschritt eine Hydroformylierung und/oder eine Oligomerisierung durchgeführt, um den Kohlenwasserstoffstrom weiter an Olefinen abzureichern, wobei der Austragsstrom aus der Hydroformylierung und/oder der Oligomerisierung nach Abtrennung der Produktealdehyde bzw. Produktoligomere als Desorptionsstrom verwendet wird.

Die Hydroformylierung, also die Reaktion eines Olefins mit Synthesegas zur Bildung eines Aldehyds, wird insbesondere unter Verwendung eines Übergangsmetalls und optional organischen Phosphor-haltigen Liganden durchgeführt. Als Übergangsmetall können Eisen, Ruthenium, Iridium, Cobalt oder Rhodium, vorzugsweise Cobalt oder Rhodium eingesetzt werden. Der Druck bei der Hydroformylierung kann zwischen 10 und 400 bar, vorzugsweise 15 bis 250 bar betragen. Die Temperatur bei der Hydroformylierung beträgt vorzugsweise 70 bis 250 °C, vorzugsweise 100 bis 200 °C.

Die Oligomerisierung kann bei einer Temperatur im Bereich von 50 bis 200 °C, vorzugsweise 60 bis 180 °C, bevorzugt im Bereich von 60 bis 130°C, und bei einem Druck von 10 bis 70 bar, bevorzugt von 20 bis 55 bar. Sofern die Oligomerisierung in flüssiger Phase erfolgen soll, müssen die Parameter Druck und Temperatur hierfür so gewählt werden, dass der Eduktstrom (die eingesetzten Olefine oder Olefingemische) in flüssiger Phase vorliegt. Als Katalysatoren für die Oligomerisierung können insbesondere Übergangsmetalle wie Nickel auf einem amorphen oder kristallinen Trägermaterial aus Aluminiumoxid, Siliciumoxid oder Alumosilicat eingesetzt werden.

Der wie vorgenannt beschrieben erhaltene Desorptionsstrom kann flüssig oder gasförmig sein und in flüssiger Phase oder in der Gasphase über den Adsorber im Desorptionsmodus geleitet werden. Bevorzugt ist der Desorptionsstrom in der Gasphase. Die Temperatur, bei der der Desorptionsstrom über den mindestens einen Adsorber im Desorptionsmodus geleitet wird, ist vorzugsweise höher als die Temperatur, bei der der zu reinigende Kohlenwasserstoffstrom über den mindestens einen Adsorber im Adsorptionsmodus geleitet wird. Dadurch wird sichergestellt, dass möglichst viele der adsorbierten Verunreinigungen wieder ausgetragen werden und der Adsorber möglichst umfassend regeneriert wird. Die Temperatur im Desorptionsmodus beträgt 280 bis 350 °C, vorzugsweise 290 bis 320 °C. Der Druck im Desorptionsmodus kann 2 bis 4 bar, vorzugsweise 2,5 bis 3,5 bar betragen.

Der Desorptionsstrom ist nach dem Überleiten über den Adsorber mit den Verunreinigungen, die er vom Adsorber aufgenommen hat kontaminiert. Der kontaminierte Desorptionsstrom kann dann als Co-Feed bei Cracking-Verfahren, als Co-Feed bei der Synthesegaserzeugung, als LPG (liquefied petroleum gas) oder als Brenngas verwendet werden. Grundsätzlich wäre es aber auch möglich, dass der kontaminierte Desorptionsstrom dann auch über einen Adsorber, beispielsweise einen irreversiblen Adsorber (wird nach vollständiger Beladung entsorgt) geleitet und von den Kontaminanten, also den im kontaminierten Desorptionsstrom enthaltenen Stickstoff-, Sauerstoff- und/oder Schwefel-haltigen Verunreinigungen, befreit wird, um nachfolgend in einem weiteren Verfahrensschritt, insbesondere einer Alkylierung, einer Oligomerisierung oder einer Hydroformylierung, eingesetzt zu werden.

In einer bevorzugten Ausführungsform können in dem erfindungsgemäßen Verfahren mindestens drei Adsorber verwendet werden, wobei sich immer zwei Adsorber im Adsorptionsmodus und ein Adsorber im Desorptionsmodus oder umgekehrt, d. h. sich immer zwei Adsorber im Desorptionsmodus und ein Adsorber im Adsorptionsmodus, befinden. Auch in dieser Ausführungsform befinden sich die Adsorber abwechselnd im Adsorptions- und im Desorptionsmodus, mit der Maßgabe, dass zwei Adsorber sich im gleichen Modus befinden. Wenn sich bei der vorgenannten Ausführungsform zwei Adsorber im Adsorptionsmodus oder im Desorptionsmodus befinden, können die Adsorber hintereinander (in Reihe geschaltet) oder nebeneinander (parallelgeschaltet) betrieben werden. Die Ausführungsform mit drei Adsorbern, bei der sich zwei Adsorber, die hintereinander oder nebeneinander betrieben werden, im Adsorptionsmodus befinden, ist bevorzugt. Die drei Adsorber sind jeweils ausgewählt aus den vorgenannten Materialien und können gleich oder verschieden voneinander sein.

In einer weiterhin bevorzugten Ausführungsform der vorliegenden Erfindung werden in dem Verfahren zur Reinigung von Kohlenwasserstoffströmen mindestens 4 Adsorber verwendet, wobei zwei Adsorber im Adsorptionsmodus und sich je ein Adsorber im Desorptionsmodus und ein Adsorber im Stand-By-Modus befinden. Die Adsorber befinden sich insbesondere abwechselnd im Adsorptions-, im Desorptions- und im Stand-By-Modus, mit der Maßgabe, dass sich immer zwei Adsorber sich im Adsorptionsmodus befinden. Die beiden Adsorber im Adsorptionsmodus werden dabei hintereinandergeschaltet betrieben. Die vier Adsorber sind jeweils ausgewählt aus den vorgenannten Materialien und können gleich oder verschieden voneinander sein.

Im Stand-By-Modus ist ein Adsorber, der vorher desorbiert oder neu eingesetzt wurde, und sich aktuell weder im Desorptionsmodus noch im Adsorptionsmodus befindet. Der Adsorber wird stehen gelassen und kann bei Bedarf in den Adsorptionsmodus überführt werden. Der Adsorber wird vorzugsweise bei Umgebungstemperatur stehen gelassen. Der Adsorber ist vorzugsweise in einem druck- und temperaturbeständigen Behälter, insbesondere einem Reaktor, angeordnet. Der Behälter ist vorzugsweise mit Desorptionsstrom gefüllt, um mögliche Reaktionen oder Ablagerungen zu vermeiden. Der Druck beträgt insbesondere 1,5 bis 5 bar. Der Desorptionsstrom liegt vorzugsweise flüssig im Stand-By-Modus im Behälter vor. Bei einem Wechsel vom Stand-By-Modus in den Adsorptionsmodus kann der vorhandene Desorptionsstrom dann sukzessive durch den zu reinigenden Kohlenwasserstoffstrom verdrängt werden.

Das erfindungsgemäße Verfahren umfasst auch den Wechsel eines Adsorbers von dem Adsorptionsmodus in den Desorptionsmodus und umgekehrt. Ein Adsorber hat naturgemäß keine unbegrenzte Kapazität zur Aufnahme bzw. Adsorption von Verunreinigungen, sondern erreicht irgendwann seine maximale Aufnahmekapazität und kann dann keine weiteren Verunreinigungen mehr adsorbieren. Spätestens zu diesem Zeitpunkt, vorzugsweise schon im Voraus muss der Adsorber ausgetauscht werden, um zu verhindern, dass ein verunreinigter Kohlenwasserstoffstrom zum nachgeschalteten Verfahrensschritt gelangt. Ein Austausch des Adsorbers wäre allein aus wirtschaftlichen Gründen unvorteilhaft. Stattdessen werden nach dem erfindungsgemäßen Verfahren mindestens zwei Adsorber eingesetzt, die abwechselnd im Adsorptions- und im Desorptionsmodus sind, so dass der verunreinigte, zu reinigende Kohlenwasserstoffstrom immer über einen Adsorber geleitet wird, der eine ausreichende Kapazität aufweist, um Verunreinigungen aufzunehmen.

Der Wechsel eines Adsorbers erfolgt vorzugsweise durch eine Steuerung des Durchflusses, insbesondere eine Ventilfolge. Dadurch kann der Zufluss des zu reinigenden Kohlenwasserstoffstroms zum Adsorber, der sich aktuell noch im Adsorptionsmodus befindet, vollständig unterbunden werden, während gleichzeitig der Zufluss des zu reinigenden Kohlenwasserstoffstroms zum Adsorber, der sich im Desorptionsmodus befindet bzw. befand, geöffnet wird, so dass der gesamte zu reinigende Kohlenwasserstoffstrom über diesen Adsorber fließt. Gleiches gilt für den Desorptionsstrom. Nach dem Wechsel wird der zu reinigende Kohlenwasserstoffstrom also über den Adsorber geleitet, der sich vor dem Wechsel im Desorptionsmodus befunden hat und der Desorptionsstrom wird nach dem Wechsel über den Adsorber geleitet, der sich vor dem Wechsel im Adsorptionsmodus befunden hat. Sobald der Kohlenwasserstoffstrom nach dem Wechsel über den gerade in den Adsorptionsmodus gewechselten Adsorber strömt, wird dieser gereinigte Kohlenwasserstoffstrom zum nachgeschalteten Verfahrensschritt geleitet. Dementsprechende anlagentechnische Aufbauten und Verschaltungen sind dem Fachmann geläufig.

Eine alternative Ausführung des Wechsels vom Desorptionsmodus in den Adsorptionsmodus ist, dass der Zufluss des zu reinigenden Kohlenwasserstoffstroms (bei gleichzeitiger Abschaltung des Zuflusses an Desorptionsstrom) auf den nächsten Adsorber umgestellt wird, bis der gesamte zu reinigende Kohlenwasserstoffstrom über diesen Adsorber fließt, der damit in den Adsorptionsmodus überführt wird. Um zu verhindern, dass hohe Mengen an inerten Alkanen, die sich durch die vorherige Desorption mit dem Desorptionsstrom noch im Adsorber befinden, zum mindestens einen Verfahrensschritt gelangen, wird der gereinigte Strom zunächst wie der kontaminierte Desorptionsstrom ausgeschleust. Erst nach einer gewissen Zeit, die kleiner der rechnerisch maximalen Verweilzeit in dem Adsorber bei gegebenem Volumenstrom des zu reinigenden Stroms ist, wird der gereinigte Strom dann zum mindestens einen Verfahrensschritt geführt.

Der Wechsel zwischen Adsorptionsmodus und Desorptionsmodus kann gesteuert werden, um zu erreichen, dass der Adsorber im Adsorptionsmodus bereits vor dem Erreichen der maximalen Aufnahmekapazität in den Desorptionsmodus überführt wird. Der Wechsel eines Adsorbers von dem Adsorptionsmodus in den Desorptionsmodus erfolgt dabei vorzugsweise nach einer bestimmten Zeit, die sich der Adsorber im Adsorptionsmodus befunden hat, nach einer bestimmten Menge an Verunreinigungen, die in dem über den Adsorber geleiteten Kohlenwasserstoffstrom vorhanden waren, oder nach einer bestimmten Menge an zu reinigendem Kohlenwasserstoffstrom, der über den Adsorber geleitet worden ist. Eine weitere Möglichkeit ist, dass nach dem ersten Adsorber die Menge an Verunreinigungen in dem Kohlenwasserstoffstrom, der über den ersten Adsorber geleitet worden ist, gemessen und bei Überschreiten eines bestimmten Grenzwertes an Verunreinigungen der Wechsel vom Adsorptionsmodus in den Desorptionsmodus erfolgt.

Sofern drei Adsorber vorhanden sind, wechseln die einzelnen Adsorber ebenfalls zwischen Adsorptions- und Desorptionsmodus. Die genaue Reihenfolge des Wechsels vom Adsorptionsmodus in den Desorptionsmodus (oder umgekehrt) für die einzelnen Adsorber ist abhängig von Aufbau und Betriebsweise. Dabei ist nur zu beachten, dass kein Adsorber im Adsorptionsmodus seine maximale Aufnahmekapazität erreicht, sondern bereits davor ein Wechsel in den Desorptionsmodus erfolgt. Der Wechsel kann bei Vorliegen von mindestens drei Adsorbern nach den bereits beschriebenen Mechanismen (bspw. Zeit, Menge) gesteuert werden.

Für den Fall (bei Vorliegen von mindestens drei Adsorbern), dass sich zwei Adsorber im Adsorptionsmodus und einer im Desorptionsmodus befinden, wobei die beiden Adsorber im Adsorptionsmodus hintereinander, also in Reihe geschaltet vorliegen, läuft der Wechsel wie folgt ab:
Der (in der Reihenschaltung) erste Adsorber im Adsorptionsmodus, also der Adsorber, der als erstes mit dem zu reinigenden Kohlenwasserstoffstrom in Kontakt kommt und daher den größten Teil der Verunreinigungen adsorbiert, befindet sich nach dem Wechsel im Desorptionsmodus. Der in der Reihenschaltung zweite Adsorber im Adsorptionsmodus fungiert nach dem Wechsel als erster Adsorber und kommt als erstes mit dem zu reinigenden Kohlenwasserstoffstrom in Kontakt kommt. Der Adsorber, der sich vorher im Desorptionsmodus befunden hat, ist nach dem Wechsel der (in der Reihenschaltung) zweite Adsorber im Adsorptionsmodus.

In der Ausführungsform mit mindestens vier Adsorbern kommt noch hinzu, dass sich - neben zwei Adsorbern im Adsorptionsmodus und einem Adsorber im Desorptionsmodus - ein Adsorber im Stand-By-Modus befindet und auf Abruf mit dem zu reinigenden Kohlenwasserstoffstrom befahren werden kann. Dadurch ergibt sich, dass sich zwei Adsorber im Adsorptionsmodus befinden und hintereinander, also in Reihe geschaltet, betrieben werden, und sich je ein Adsorber im Desorptionsmodus und ein vorher desorbierter oder neu eingesetzter Adsorber im Stand-By-Modus befinden. Der Wechsel zwischen den einzelnen Modi, insbesondere zwischen Adsorptions- und Desorptions- und Stand-By-Modus läuft wie folgt ab:
Der (in der Reihenschaltung) erste Adsorber im Adsorptionsmodus, also der Adsorber, der als erstes mit dem zu reinigenden Kohlenwasserstoffstrom in Kontakt kommt und daher den größten Teil der Verunreinigungen adsorbiert, befindet sich nach dem Wechsel im Desorptionsmodus. Der (in der Reihenschaltung) zweite Adsorber im Adsorptionsmodus fungiert nach dem Wechsel als (in der Reihenschaltung) erster Adsorber und kommt als erstes mit dem zu reinigenden Kohlenwasserstoffstrom in Kontakt. Der Adsorber, der sich vor dem Wechsel im Desorptionsmodus befindet hat, ist nach dem Wechsel im Stand-By-Modus und der Adsorber, der sich vor dem Wechsel im Stand-By-Modus befunden hat, ist nach dem Wechsel der (in der Reihenschaltung) zweite Adsorber im Adsorptionsmodus. Der Wechsel kann bei Vorliegen von mindestens vier Adsorbern nach den bereits beschriebenen Mechanismen (bspw. Zeit, Menge) gesteuert werden.

In einer besonders bevorzugten Ausführungsform betrifft die vorliegende Erfindung ein Verfahren zur Reinigung von Kohlenwasserstoffströmen, umfassend Olefine mit 4 Kohlenstoffatomen, insbesondere n-Butenen, wobei der gereinigte Kohlenwasserstoffstrom mindestens einem Verfahrensschritt, welcher eine Olefinumsetzung, welche aus der Gruppe, bestehend aus Veretherung, Hydroformylierung, Oligomerisierung, (Methoxy)Carbonylierung, Alkylierung, Metathese und Epoxidation, ausgewählt wird, und eine Produktabtrennung umfasst, unterworfen wird,
wobei in dem Verfahren mindestens zwei Adsorber verwendet werden, von denen sich ein Adsorber im Adsorptionsmodus und ein Adsorber im Desorptionsmodus befindet,
wobei im Adsorptionsmodus der zu reinigende Kohlenwasserstoffstrom über den Adsorber geleitet wird, um Stickstoff-, Sauerstoff- und/oder Schwefel-haltige Verunreinigungen aus dem zu reinigenden Kohlenwasserstoffstrom zu entfernen,
wobei im Desorptionsmodus ein Desorptionsstrom bei einer Temperatur von 280 bis 350 °C über den Adsorber geleitet wird, um die adsorbierten Verunreinigungen vom Adsorber zu desorbieren, und
dadurch gekennzeichnet, dass ein nach dem mindestens einen Verfahrensschritt entnommener und an den eingesetzten Olefinen und den bei der Olefinumsetzung gebildeten Produkten abgereicherter Kohlenwasserstoffstrom, der nicht hydriert wird und der einen Gehalt an den eingesetzten und nicht umgesetzten Olefinen von 2 bis 30 Gew.-% aufweist, als Desorptionsstrom verwendet wird.

Die vorliegende Erfindung wird nachfolgend anhand der Zeichnungen genauer erläutert. Die in den Zeichnungen dargestellten Verfahrensabläufe sind lediglich bevorzugte Ausführungsformen und sind nicht einschränkend zu verstehen.

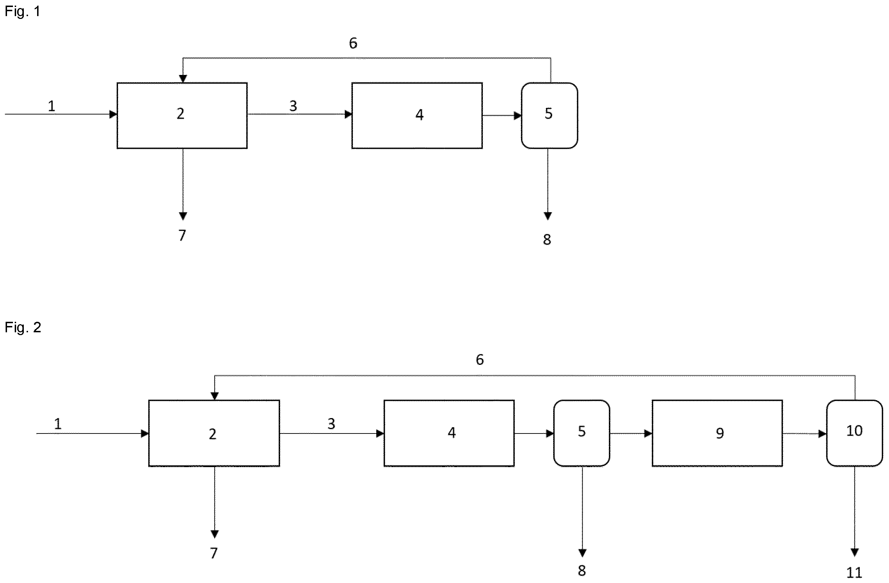

Fig. 1 zeigt eine Ausführungsform, bei der nur ein Verfahrensschritt durchgeführt wird. Dabei wird der eingesetzte Kohlenwasserstoffstrom (1) zunächst in der Adsorptions-/Desorptionszone (2) über einen Adsorber im Adsorptionsmodus geleitet und anschließend als gereinigter Kohlenwasserstoffstrom (3) zu dem einen Verfahrensschritt, der aus einer Reaktionszone (4), in der die jeweilige Umsetzung der Olefine erfolgt, und nachfolgender Produktabtrennung (5) zur Abtrennung der Produkte (8) besteht, geführt. Der aus der Produktabtrennung (5) erhaltene und an den eingesetzten Olefinen und den bei der Umsetzung gebildeten Produkten (8) abgereicherte Kohlenwasserstoffstrom wird dann als Desorptionsstrom (6) für einen Adsorber im Desorptionsmodus in der Adsorptions-/Desorptionszone (2) verwendet, wodurch ein mit den desorbierten Verunreinigungen kontaminierter Desorptionsstrom (7) erhalten wird.
Fig. 2 zeigt eine Ausführungsform, bei dem zwei Verfahrensschritte durchgeführt werden. Dabei wird der eingesetzte Kohlenwasserstoffstrom (1) zunächst in der Adsorptions-/Desorptionszone (2) über einen Adsorber im Adsorptionsmodus geleitet und anschließend als gereinigter Kohlenwasserstoffstrom (3) zur einem ersten Verfahrensschritt, der aus einer ersten Reaktionszone (4), in der die jeweilige erste Umsetzung der Olefine erfolgt, und nachfolgender erster Produktabtrennung (5) zur Abtrennung der Produkte (8) besteht, geführt. Der aus der ersten Produktabtrennung erhaltene Kohlenwasserstoffstrom wird dann einem zweiten Verfahrensschritt, der aus einer zweiten Reaktionszone (9), in der die jeweilige zweite Umsetzung der Olefine erfolgt, und nachfolgender zweiter Produktabtrennung (10) zur Abtrennung der Produkte (11) aus der zweiten Umsetzung besteht, zugeführt. Der aus der zweiten Produktabtrennung (10) erhaltene und an den eingesetzten Olefinen und den bei der Umsetzung gebildeten Produkten abgereicherte Kohlenwasserstoffstrom wird dann als Desorptionsstrom (6) für einen Adsorber im Desorptionsmodus in der Adsorptions-/Desorptionszone (2) verwendet, wodurch ein mit den desorbierten Verunreinigungen kontaminierter Desorptionsstrom (7) erhalten wird.

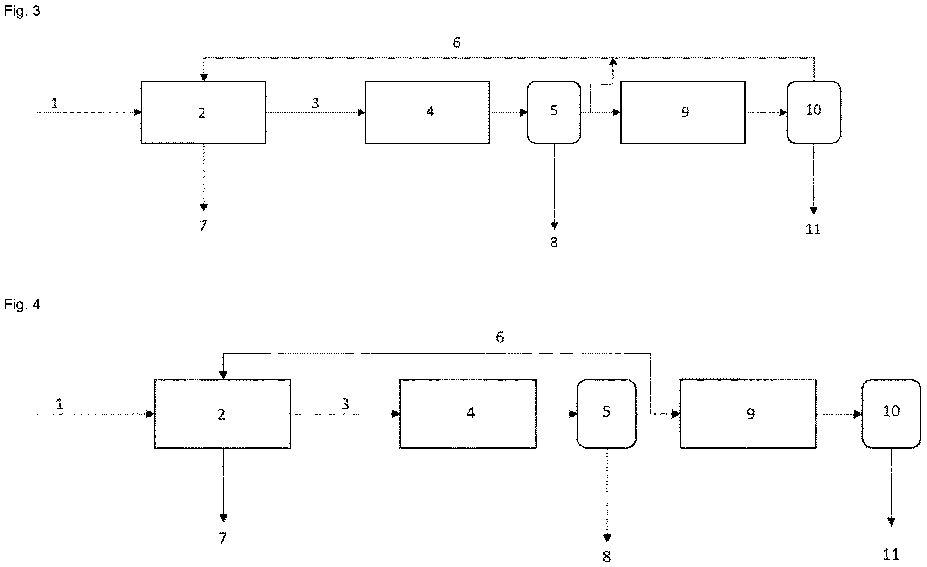

Fig. 3 zeigt eine Ausführungsform, bei dem zwei Verfahrensschritte durchgeführt werden, wobei ein Teil des aus der ersten Produktabtrennung erhaltenen Kohlenwasserstoffstroms um den zweiten Verfahrensschritt herumgeführt wird. Dabei wird der eingesetzte Kohlenwasserstoffstrom (1) zunächst in der Adsorptions-/Desorptionszone (2) über einen Adsorber im Adsorptionsmodus geleitet und anschließend als gereinigter Kohlenwasserstoffstrom (3) zu einem ersten Verfahrensschritt, der aus einer ersten Reaktionszone (4), in der die jeweilige erste Umsetzung der Olefine erfolgt, und nachfolgender erster Produktabtrennung (5) zur Abtrennung der Produkte (8) besteht, geführt. Der aus der ersten Produktabtrennung erhaltene Kohlenwasserstoffstrom wird dann teilweise als Bestandteil des Desorptionsstroms (6) zur Adsorptions-/Desorptionszone (2) geleitet und teilweise einem zweiten Verfahrensschritt, der aus einer zweiten Reaktionszone (9), in der die jeweilige zweite Umsetzung der Olefine erfolgt, und nachfolgender zweiter Produktabtrennung (10) zur Abtrennung der Produkte (11) aus der zweiten Umsetzung besteht, zugeführt. Der aus der zweiten Produktabtrennung (10) erhaltene und an den eingesetzten Olefinen und den bei der Umsetzung gebildeten Produkten abgereicherte Kohlenwasserstoffstrom und ein Teil des aus der ersten Produktabtrennung (5) erhaltenen Kohlenwasserstoffstrom werden gemeinsam als Desorptionsstrom (6) für einen Adsorber im Desorptionsmodus in der Adsorptions-/Desorptionszone (2) verwendet, wodurch ein mit den desorbierten Verunreinigungen kontaminierter Desorptionsstrom (7) erhalten wird.
Fig. 4 zeigt eine Ausführungsform, bei dem zwei Verfahrensschritte durchgeführt werden, wobei nur ein Teil des aus der ersten Produktabtrennung erhaltenen Kohlenwasserstoffstroms als Desorptionsstrom verwendet wird. Dabei wird der eingesetzte Kohlenwasserstoffstrom (1) zunächst in der Adsorptions-/Desorptionszone (2) über einen Adsorber im Adsorptionsmodus geleitet und anschließend als gereinigter Kohlenwasserstoffstrom (3) zur einem ersten Verfahrensschritt, der aus einer ersten Reaktionszone (4), in der die jeweilige erste Umsetzung der Olefine erfolgt, und nachfolgender erster Produktabtrennung (5) zur Abtrennung der Produkte (8) besteht, geführt. Der aus der ersten Produktabtrennung erhaltene Kohlenwasserstoffstrom wird dann teilweise um den zweiten Verfahrensschritt herumgeführt und teilweise einem zweiten Verfahrensschritt, der aus einer zweiten Reaktionszone (9), in der die jeweilige zweite Umsetzung der Olefine erfolgt, und nachfolgender zweiter Produktabtrennung (10) zur Abtrennung der Produkte (11) aus der zweiten Umsetzung besteht, zugeführt. Der um den zweiten Verfahrensschritt herumgeführte Teil des aus der ersten Produktabtrennung (5) erhaltenen Kohlenwasserstoffstroms wird dann als Desorptionsstrom (6) für einen Adsorber im Desorptionsmodus in der Adsorptions-/Desorptionszone (2) verwendet, wodurch ein mit den desorbierten Verunreinigungen kontaminierter Desorptionsstrom (7) erhalten wird.

## Patentansprüche

1. Verfahren zur Reinigung von Kohlenwasserstoffströmen, umfassend Olefine mit 2 bis 8 Kohlenstoffatomen, wobei der gereinigte Kohlenwasserstoffstrom mindestens einem Verfahrensschritt, welcher eine Olefinumsetzung, welche aus der Gruppe, bestehend aus Veretherung, Hydroformylierung, Oligomerisierung, (Methoxy)Carbonylierung, Alkylierung, Metathese und Epoxidation, ausgewählt wird, und eine Produktabtrennung umfasst, unterworfen wird,
wobei in dem Verfahren mindestens zwei Adsorber verwendet werden, von denen sich ein Adsorber im Adsorptionsmodus und ein Adsorber im Desorptionsmodus befindet,
wobei im Adsorptionsmodus der zu reinigende Kohlenwasserstoffstrom über den Adsorber geleitet wird, um Stickstoff-, Sauerstoff- und/oder Schwefel-haltige Verunreinigungen aus dem zu reinigenden Kohlenwasserstoffstrom zu entfernen,
wobei im Desorptionsmodus ein Desorptionsstrom bei einer Temperatur von 280 bis 350 °C über den Adsorber geleitet wird, um die adsorbierten Verunreinigungen vom Adsorber zu desorbieren, und
**dadurch gekennzeichnet, dass** ein nach dem mindestens einen Verfahrensschritt entnommener und an den eingesetzten Olefinen und den bei der Olefinumsetzung gebildeten Produkten abgereicherter Kohlenwasserstoffstrom, der nicht hydriert wird und der einen Gehalt an den eingesetzten und nicht umgesetzten Olefinen von 2 bis 30 Gew.-% aufweist, als Desorptionsstrom verwendet wird.

2. Verfahren nach Anspruch 1, wobei der nach dem mindestens einen Verfahrensschritt entnommener und an den eingesetzten Olefinen und den bei der Olefinumsetzung gebildeten Produkten abgereicherter Kohlenwasserstoffstrom einen Gehalt an Produkten von maximal 500 Gewichts-ppm, vorzugsweise maximal 350 Gewichts-ppm, besonders bevorzugt maximal 170 Gewichts-ppm aufweist.

3. Verfahren nach Anspruch 1 oder 2, wobei der der eingesetzte Kohlenwasserstoffstrom einen Gehalt an Olefinen von 35 bis 95 Gew.-% aufweist.

4. Verfahren nach einem der Ansprüche 1 bis 3, wobei der Wechsel eines Adsorbers vom Adsorptionsmodus in den Desorptionsmodus so erfolgt, dass der zu reinigende Kohlenwasserstoffstrom nach dem Wechsel über den Adsorber geleitet wird, der sich vor dem Wechsel im Desorptionsmodus befunden hat und der Desorptionsstrom nach dem Wechsel über den Adsorber geleitet wird, der sich vor dem Wechsel im Adsorptionsmodus befunden hat.

5. Verfahren nach Anspruch 4, wobei der Wechsel nach einer bestimmten Zeit, die sich der Adsorber im Adsorptionsmodus befunden hat, nach einer bestimmten Menge an Verunreinigungen, die in dem über den Adsorber geleiteten Kohlenwasserstoffstrom vorhanden waren und vom Adsorber aufgenommen wurden, oder nach einer bestimmten Menge an zu reinigendem Kohlenwasserstoffstrom, der über den Adsorber geleitet worden ist, erfolgt oder wobei nach dem ersten Adsorber die Menge an Verunreinigungen in dem Kohlenwasserstoffstrom, der über den ersten Adsorber geleitet worden ist, gemessen wird und bei Überschreiten eines bestimmten Grenzwertes an Verunreinigungen der Wechsel erfolgt.

6. Verfahren nach einem der Ansprüche 1 bis 5, wobei in dem Verfahren mindestens drei Adsorber verwendet werden, von denen sich zwei im Adsorptionsmodus und einer im Desorptionsmodus oder umgekehrt befinden, wobei die zwei Adsorber im gleichen Modus hintereinander (in Reihe geschaltet) oder nebeneinander (parallelgeschaltet) betrieben werden.

7. Verfahren nach Anspruch 6, wobei, wenn sich zwei Adsorber, die hintereinander (in Reihe geschaltet) vorliegen, im Adsorptionsmodus befinden, der Wechsel zwischen Adsorptions- und Desorptionsmodus so abläuft, dass
der erste Adsorber im Adsorptionsmodus, also der Adsorber, der als erstes mit dem zu reinigenden Kohlenwasserstoffstrom in Kontakt kommt, sich nach dem Wechsel im Desorptionsmodus befindet;
der zweite Adsorber im Adsorptionsmodus nach dem Wechsel als erster Adsorber fungiert und als erstes mit dem zu reinigenden Kohlenwasserstoffstrom in Kontakt kommt; und
der Adsorber, der sich vor dem Wechsel im Desorptionsmodus befunden hat, nach dem Wechsel als zweiter Adsorber im Adsorptionsmodus fungiert.

8. Verfahren nach einem der Ansprüche 1 bis 7, wobei in dem Verfahren mindestens vier Adsorber verwendet werden, von denen sich wechselnd zwei Adsorber im Adsorptionsmodus und sich je ein Adsorber im Desorptionsmodus und ein Adsorber im Stand-By-Modus befinden,
wobei die zwei Adsorber, die sich im Adsorptionsmodus befinden, hintereinander (in Reihe geschaltet) betrieben werden,
wobei der Adsorber im Stand-By-Modus ein Adsorber ist, der vorher desorbiert wurde oder neu eingesetzt wurde, und im Stand-By-Modus bis zum nächsten Wechsel stehen gelassen wird,
wobei der Wechsel zwischen Adsorptions- und Desorptions- und Stand-By-Modus so abläuft, dass der erste Adsorber im Adsorptionsmodus, also der Adsorber, der als erstes mit dem zu reinigenden Kohlenwasserstoffstrom in Kontakt kommt, sich nach dem Wechsel im Desorptionsmodus befindet;
der zweite Adsorber im Adsorptionsmodus nach dem Wechsel als erster Adsorber fungiert und als erstes mit dem zu reinigenden Kohlenwasserstoffstrom in Kontakt kommt;
der Adsorber im Desorptionsmodus sich nach dem Wechsel im Stand-By-Modus befindet; und
der Adsorber im Stand-By-Modus nach dem Wechsel als zweiter Adsorber im Adsorptionsmodus fungiert.

9. Verfahren nach einem der Ansprüche 1 bis 8, wobei der zu reinigende Kohlenwasserstoffstrom in flüssiger Phase über den mindestens einen Adsorber im Adsorptionsmodus geleitet wird.

10. Verfahren nach einem der Ansprüche 1 bis 9, wobei der zu reinigende Kohlenwasserstoffstrom bei einer Temperatur im Bereich von 0 bis 60 °C, vorzugsweise 10 bis 50 °C, besonders bevorzugt 20 bis 40 °C über den mindestens einen Adsorber im Adsorptionsmodus geleitet wird.

11. Verfahren nach einem der der Ansprüche 1 bis 10, wobei der mit dem Verfahren zu reinigende Kohlenwasserstoffstrom Olefine mit 4 Kohlenstoffatomen umfasst.

12. Verfahren nach einem der Ansprüche 1 bis 11, wobei der Desorptionsstrom im Desorptionsmodus in entgegengesetzter Richtung zur Strömungsrichtung des zu reinigen Kohlenwasserstoffstroms im Adsorptionsmodus über den Adsorber geleitet wird.

13. Verfahren nach einem der Ansprüche 1 bis 12, wobei der kontaminierte Desorptionsstrom, welcher über den Adsorber im Desorptionsmodus geleitet worden ist, als Co-Feed bei Cracking-Verfahren, als Co-Feed bei der Synthesegaserzeugung, als LPG (liquefied petroleum gas) oder als Brenngas verwendet wird.

14. Verfahren nach einem der Ansprüche 1 bis 12, wobei der kontaminierte Desorptionsstrom über einen Adsorber geleitet und von den Kontaminanten befreit wird, um nachfolgend in einem weiteren Verfahrensschritt, insbesondere einer Alkylierung, einer Oligomerisierung oder einer Hydroformylierung, eingesetzt zu werden.

15. Verfahren nach Anspruch 14, wobei der Adsorber ein irreversibler Adsorber ist.

## Claims

1. Process for purifying hydrocarbon streams comprising olefins having 2 to 8 carbon atoms, wherein the purified hydrocarbon stream is subjected to at least one process step comprising an olefin conversion selected from the group consisting of etherification, hydroformylation, oligomerization, (methoxy)carbonylation, alkylation, metathesis and epoxidation and a product separation,
wherein the process employs at least two adsorbers of which one adsorber is in adsorption mode and one adsorber is in desorption mode,
wherein in adsorption mode the hydrocarbon stream to be purified is passed through the adsorber to remove nitrogen-, oxygen- and/or sulfur-containing contaminants from the hydrocarbon stream to be purified,
wherein in desorption mode a desorption stream is passed through the adsorber at a temperature of 280°C to 350°C to desorb the adsorbed contaminants from the adsorber, and
**characterized in that** a hydrocarbon stream withdrawn after the at least one process step and depleted in the employed olefins and the products formed in the olefin conversion which is not hydrogenated and which has a content of the employed and unconverted olefins of 2% to 30% by weight is used as the desorption stream.

2. Process according to Claim 1, wherein the hydrocarbon stream withdrawn after the at least one process step and depleted in the employed olefins and the products formed in the olefin conversion has a content of products of not more than 500 ppm by weight, preferably not more than 350 ppm by weight, particularly preferably not more than 170 ppm by weight.

3. Process according to Claim 1 or 2, wherein the employed hydrocarbon stream has a content of olefins of 35% to 95% by weight.

4. Process according to any of Claims 1 to 3, wherein the switchover of an adsorber from adsorption mode to desorption mode is carried out such that after the switchover the hydrocarbon stream to be purified is passed through the adsorber which was in desorption mode before the switchover and after the switchover the desorption stream is passed through the adsorber which was in adsorption mode before the switchover.

5. Process according to Claim 4, wherein the switchover is carried out after a certain time for which the adsorber has been in adsorption mode, after a certain amount of contaminants present in the hydrocarbon stream passed through the adsorber have been taken up by the adsorber, or after a certain amount of hydrocarbon stream to be purified has been passed through the adsorber or wherein the amount of contaminants in the hydrocarbon stream passed through the first adsorber is measured downstream of the first adsorber and the switchover is carried out upon exceedance of a particular threshold value of contaminants.

6. Process according to any of Claims 1 to 5, wherein the process employs at least three adsorbers of which two are in adsorption mode and one is in desorption mode or vice versa, wherein the two adsorbers in the same mode are operated in series or in parallel.

7. Process according to Claim 6, wherein, when two adsorbers arranged in series are in adsorption mode, the switchover between adsorption and desorption mode proceeds such that
the first adsorber in adsorption mode, i.e. the adsorber that is first to come into contact with the hydrocarbon stream to be purified, is in desorption mode after the switchover;
the second adsorber in adsorption mode functions as the first adsorber after the switchover and is the first to come into contact with the hydrocarbon stream to be purified; and
the adsorber that was in desorption mode before the switchover functions as the second adsorber in adsorption mode after the switchover.

8. Process according to any of Claims 1 to 7, wherein the process employs at least four adsorbers of which alternately two adsorbers are in adsorption mode and one adsorber is in desorption mode while one adsorber is in standby mode,
wherein the two adsorbers in adsorption mode are operated in series,
wherein the adsorber in standby mode is a previously desorbed or newly installed adsorber and is left in standby mode until the next switchover,
wherein the switchover between adsorption and desorption and standby mode proceeds such that the first adsorber in adsorption mode, i.e. the adsorber that is first to come into contact with the hydrocarbon stream to be purified, is in desorption mode after the switchover; the second adsorber in adsorption mode functions as the first adsorber after the switchover and is the first to come into contact with the hydrocarbon stream to be purified;
the adsorber in desorption mode is in standby mode after the switchover; and
the adsorber in standby mode functions as the second adsorber in adsorption mode after the switchover.

9. Process according to any of Claims 1 to 8, wherein the hydrocarbon stream to be purified is passed through the at least one adsorber in the liquid phase in adsorption mode.

10. Process according to any of Claims 1 to 9, wherein the hydrocarbon stream to be purified is passed through the at least one adsorber in adsorption mode at a temperature in the range from 0°C to 60°C, preferably 10°C to 50°C, particularly preferably 20°C to 40°C.

11. Process according to any of Claims 1 to 10, wherein the hydrocarbon stream to be purified with the process comprises olefins having 4 carbon atoms.

12. Process according to any of Claims 1 to 11, wherein the desorption stream in desorption mode is passed through the adsorber in the opposite direction to the flow direction of the hydrocarbon stream to be purified in adsorption mode.

13. Process according to any of Claims 1 to 12, wherein the contaminated desorption stream that has been passed through the adsorber in desorption mode is used as co-feed in cracking processes, as co-feed in synthesis gas generation, as LPG (liquefied petroleum gas) or as fuel gas.

14. Process according to any of Claims 1 to 12, wherein the contaminated desorption stream is passed through an adsorber and freed from the contaminants in order to subsequently be used in a further process step, in particular an alkylation, an oligomerization or a hydroformylation.

15. Process according to Claim 14, wherein the adsorber is an irreversible adsorber.

## Revendications

1. Procédé pour la purification de flux hydrocarbonés, comprenant des oléfines comportant 2 à 8 atomes de carbone, le flux hydrocarboné purifié étant soumis à au moins une étape de procédé, qui comprend une transformation d'oléfines, qui est choisie dans le groupe constitué par une éthérification, une hydroformylation, une oligomérisation, une (méthoxy)carbonylation, une alkylation, une métathèse et une époxydation, et une séparation des produits,
dans lequel au moins deux adsorbants sont utilisés dans le procédé, dont un adsorbant se trouve dans le mode d'adsorption et un adsorbant se trouve dans le mode de désorption,
dans lequel, dans le mode d'adsorption, le flux hydrocarboné à purifier est guidé sur l'adsorbant pour éliminer des impuretés, contenant de l'azote, de l'oxygène et/ou du soufre, du flux hydrocarboné à purifier,
dans lequel, dans le mode de désorption, un flux de désorption est guidé sur l'adsorbant à une température de 280 à 350°C pour désorber les impuretés adsorbées de l'adsorbant et
**caractérisé en ce qu'**un flux hydrocarboné prélevé après ladite au moins une étape de procédé et appauvri en oléfines utilisées et en produits formés lors de la transformation d'oléfines, qui n'est pas hydrogéné et qui présente une teneur en oléfines utilisées et non transformées de 2 à 30% en poids, est utilisé comme flux de désorption.

2. Procédé selon la revendication 1, dans lequel le flux hydrocarboné prélevé après ladite au moins une étape de procédé et appauvri en oléfines utilisées et en produits formés lors de la transformation d'oléfines, présente une teneur en produits de maximum 500 ppm en poids, de préférence de maximum 350 ppm en poids, de manière particulièrement préférée de maximum 170 ppm en poids.

3. Procédé selon la revendication 1 ou 2, dans lequel le flux hydrocarboné utilisé présente une teneur en oléfines de 35 à 95% en poids.

4. Procédé selon l'une des revendications 1 à 3, dans lequel le changement d'un adsorbant à partir du mode d'adsorption dans le mode de désorption a lieu de manière telle que le flux hydrocarboné à purifier est guidé, après le changement, sur l'adsorbant qui se trouvait dans le mode de désorption avant le changement et le flux de désorption est guidé, après le changement, sur l'adsorbant qui se trouvait dans le mode d'adsorption avant le changement.

5. Procédé selon la revendication 4, dans lequel le changement a lieu après un temps déterminé, pendant lequel l'adsorbant s'est trouvé dans le mode d'adsorption, après une certaine quantité d'impuretés, qui étaient présentes dans le flux hydrocarboné guidé sur l'adsorbant et qui ont été reprises par l'adsorbant, ou après une quantité déterminée de flux hydrocarboné à purifier, qui a été guidée sur l'adsorbant, ou dans lequel la quantité d'impuretés dans le flux hydrocarboné qui a été guidé sur le premier adsorbant est mesurée et le changement a lieu lors du dépassement d'une valeur limite déterminée d'impuretés.

6. Procédé selon l'une des revendications 1 à 5, dans lequel au moins trois adsorbants sont utilisés dans le procédé, dont deux se trouvent dans le mode d'adsorption et un dans le mode de désorption ou inversement, les deux adsorbants dans le même mode étant exploités successivement (commutés en série) ou l'un à côté de l'autre (commutés en parallèle).

7. Procédé selon la revendication 6, dans lequel, lorsque deux adsorbants, qui sont situés l'un derrière l'autre (commutés en série), se trouvent dans le mode d'adsorption, le changement entre le mode d'adsorption et le mode de désorption se déroule de manière telle que le premier adsorbant dans le mode d'adsorption, c'est-à-dire l'adsorbant qui entre en contact en premier lieu avec le flux hydrocarboné à purifier, se trouve, après le changement, dans le mode de désorption ;
le deuxième adsorbant dans le mode d'adsorption fonctionne, après le changement, comme premier adsorbant et entre en contact en premier lieu avec le flux hydrocarboné à purifier ; et
l'adsorbant qui se trouvait avant le changement dans le mode de désorption fonctionne, après le changement, comme deuxième adsorbant dans le mode d'adsorption.

8. Procédé selon l'une des revendications 1 à 7, dans lequel au moins quatre adsorbants sont utilisés dans le procédé, dont, en alternance, deux adsorbants se trouvent dans le mode d'adsorption et respectivement un adsorbant se trouve dans le mode de désorption et un adsorbant se trouve dans le mode de veille,
les deux adsorbants qui se trouvent dans le mode d'adsorption étant exploités l'un derrière l'autre (commutés en série),
l'adsorbant qui se trouve dans le mode de veille étant un adsorbant qui a été désorbé au préalable ou qui a été nouvellement utilisé et qui est laissé dans le mode de veille jusqu'au prochain changement,
le changement entre le mode de d'adsorption, le mode de désorption et le mode de veille se déroulant de manière telle que le premier adsorbant dans le mode d'adsorption, c'est-à-dire l'adsorbant qui entre en contact en premier lieu avec le fluide hydrocarboné à purifier, se trouve après le changement dans le mode de désorption ;
le deuxième adsorbant dans le mode d'adsorption fonctionne, après le changement, comme premier adsorbant et entre en contact en premier lieu avec le fluide hydrocarboné à purifier ;
l'adsorbant dans le mode de désorption se trouve, après le changement, dans le mode de veille ; et
l'adsorbant dans le mode de de veille fonctionne, après le changement, comme deuxième adsorbant dans le mode d'adsorption.

9. Procédé selon l'une des revendications 1 à 8, dans lequel le flux hydrocarboné à purifier est guidé en phase liquide sur ledit au moins un adsorbant dans le mode d'adsorption.

10. Procédé selon l'une des revendications 1 à 9, dans lequel le flux hydrocarboné à purifier est guidé à une température dans la plage de 0 à 60°C, de préférence de 10 à 50°C, de manière particulièrement préférée de 20 à 40°C sur ledit au moins un adsorbant dans le mode d'adsorption.

11. Procédé selon l'une des revendications 1 à 10, dans lequel le flux hydrocarboné à purifier par le procédé comprend des oléfines comportant 4 atomes de carbone.

12. Procédé selon l'une des revendications 1 à 11, dans lequel le flux de désorption dans le mode de désorption est guidé sur l'adsorbant dans le sens opposé au sens d'écoulement du flux hydrocarboné à purifier dans le mode d'adsorption.

13. Procédé selon l'une des revendications 1 à 12, dans lequel le flux de désorption contaminé, qui a été guidé sur d'adsorbant dans le mode de désorption, est utilisé comme co-alimentation lors de procédés de craquage, comme co-alimentation lors de la production de gaz de synthèse, comme LPG (gaz de pétrole liquéfié) ou comme gaz de combustion.

14. Procédé selon l'une des revendications 1 à 12, dans lequel le flux de désorption contaminé est guidé sur un adsorbant et libéré des contaminants pour ensuite être utilisé dans une autre étape de procédé, en particulier une alkylation, une oligomérisation ou une hydroformylation.

15. Procédé selon la revendication 14, dans lequel l'adsorbant est un adsorbant irréversible.
